(19) Europäisches Patentamt
European Patent Office
Office européen des brevets

(11) **EP 2 851 431 B1**

(12) **EUROPEAN PATENT SPECIFICATION**

(45) Date of publication and mention of the grant of the patent:
**13.12.2017 Bulletin 2017/50**

(51) Int Cl.:
***C12Q 1/68*** (2006.01)

(21) Application number: **12876917.1**

(22) Date of filing: **14.05.2012**

(86) International application number:
**PCT/CN2012/075478**

(87) International publication number:
**WO 2013/170429 (21.11.2013 Gazette 2013/47)**

(54) **METHOD, SYSTEM AND COMPUTER READABLE MEDIUM FOR DETERMINING BASE INFORMATION IN PREDETERMINED AREA OF FETUS GENOME**

VERFAHREN, SYSTEM UND COMPUTERLESBARES MEDIUM ZUR BESTIMMUNG EINER BASISINFORMATION IN EINEM BESTIMMTEN FÖTUSGENOMBEREICH

PROCÉDÉ, SYSTÈME ET SUPPORT LISIBLE PAR UN ORDINATEUR DESTINÉS À DÉTERMINER DES INFORMATIONS RELATIVES AUX BASES DANS UNE ZONE PRÉDÉTERMINÉE D'UN GÉNOME FOETAL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**25.03.2015 Bulletin 2015/13**

(73) Proprietor: **BGI Genomics Co., Ltd.**
**Yantian District**
**Shenzhen, Guangdong Province 518083 (CN)**

(72) Inventors:
 • **CHEN, Shengpei**
 **Shenzhen**
 **Guangdong 518083 (CN)**
 • **GE, Huijuan**
 **Shenzhen**
 **Guangdong 518083 (CN)**
 • **LI, Xuchao**
 **Shenzhen**
 **Guangdong 518083 (CN)**
 • **YI, Shang**
 **Shenzhen**
 **Guangdong 518083 (CN)**
 • **WANG, Jian**
 **Shenzhen**
 **Guangdong 518083 (CN)**
 • **WANG, Jun**
 **Shenzhen**
 **Guangdong 518083 (CN)**
 • **YANG, Huanming**
 **Shenzhen**
 **Guangdong 518083 (CN)**
 • **ZHANG, Xiuqing**
 **Shenzhen**
 **Guangdong 518083 (CN)**

(74) Representative: **Kador & Partner**
**Corneliusstraße 15**
**80469 München (DE)**

(56) References cited:
**WO-A1-2012/021149      WO-A2-2007/092473**
**CN-A- 102 127 818      CN-A- 102 317 473**
**US-A1- 2012 095 697**

 • **WINSTON TIMP ET AL: "DNA Base-Calling from a Nanopore Using a Viterbi Algorithm", BIOPHYSICAL JOURNAL, vol. 102, no. 10, 1 May 2012 (2012-05-01), pages 37-39, XP055048922, ISSN: 0006-3495, DOI: 10.1016/j.bpj.2012.04.009**
 • **MINGJIE LIN ET AL: "Base-Calling in DNA Pyrosequencing with Reconfigurable Bayesian Network", RECONFIGURABLE COMPUTING AND FPGAS, 2009. RECONFIG '09. INTERNATIONAL CONFERENCE ON, IEEE, PISCATAWAY, NJ, USA, 9 December 2009 (2009-12-09), pages 95-100, XP031611479, ISBN: 978-1-4244-5293-4**

**(Cont. next page)**

- KUO-CHING LIANG ET AL: "Bayesian Basecalling for DNA Sequence Analysis Using Hidden Markov Models", IEEE/ACM TRANSACTIONS ON COMPUTATIONAL BIOLOGY AND BIOINFORMATICS, IEEE SERVICE CENTER, NEW YORK, NY, US, vol. 4, no. 3, 1 July 2007 (2007-07-01), pages 430-440, XP011190096, ISSN: 1545-5963, DOI: 10.1109/TCBB.2007.1027

- KUO-CHING LIANG ET AL: "Bayesian Basecalling for DNA Sequence Analysis using Hidden Markov Models", INFORMATION SCIENCES AND SYSTEMS, 2006 40TH ANNUAL CONFERENCE ON, IEEE, PI, 1 March 2006 (2006-03-01), pages 1599-1604, XP031012077, ISBN: 978-1-4244-0349-3

**Description**

TECHNICAL FIELD

The present invention generally relates to a method of determining base information of a predetermined region in a fetal genome, and a system and a computer readable medium thereof.

BACKGROUND

Genetic diseases are one kind of diseases caused by changes of genetic materials, having characteristics of being congenital, familial, permanent and hereditary. The genetic diseases may be categorized into 3 classes: monogenetic disease, polygenetic disorder and chromosome abnormality. In which the monogenetic disease is mostly because of genetic function abnormality caused by dominant or recessive inheritance of a single disease-causing gene; while the polygenetic disorder is a kind of disease caused by a plurality of gene changes, which may be influenced by external environment to some extent; and the chromosome abnormality includes number abnormality and structure abnormality, with a most common example being as a Down's Syndrome resulting from Trisomy 21, of which a child patient presenting congenital traits such as mongolism and abnormal body shape, etc. Since there are no effective therapeutic treatments for genetic diseases so far, it can only pertinently perform supportive treatments or drug remission with expensive cost, which may bring heavy burdens both in economy and spirit for societies and families. Thus, it is extremely necessary to do some preventive work by detecting pathological status with a fetus before birth, to achieve a purpose of good prenatal and postnatal care. The fetus itself is not used in this invention.

However, related detection method still needs to be improved.

WO 2007/092473 A2 discloses non-invasive fetal genetic screening by digital analysis involving the analysis of maternal blood for a genetic condition wherein mixed fetal and maternal DNA in the maternal blood is analyzed to distinguish fetal mutation or genetic abnormality from the background of the maternal DNA.

US 2012/0095697 A1 discloses a method for determining the copy number of a genomic region at a detection position of a target sequence in a sample wherein sequence coverage bias is corrected and may be normalized against a base line sample. Hidden Markov Model (HMM) segmentation, scoring and output are performed to estimate a total copy number value and region-specific copy number value for a plurality of regions.

WO 2012/021149 A1 ferers to Hidden Markov Model and other machine-learning methods which are used together in nanopore detection with the use of channel blockaders that are event transducers or event reporters, thereby enabling nanopore transduction detection and nanopore biosensing.

W. Timp et al. in Biophysical Journal, Vol. 102, No. 10, May 2012, L37-L39 disclose nanopore-based DNA sequencing. A method is demonstrated to decode 3-bp-resolution nanopore electrical measurements into a DNA sequence using a Hidden Markov model.

Mingjie Lin et al. in International Conference on Reconfigurable Computing and FPGAs, Reconfig '09, IEEE, 2009, pp. 95 - 100 disclose a reconfigurable computing method based on dynamic Bayesian learning network is proposed for base-calling in pyrosequencing from microarray gene expression data.

Kuo-ching Liang et al. in IEEE/ACM TRANSACTIONS ON COMPUTATIONAL BIOLOGY AND BIOINFORMATICS, VOL. 4, NO. 3, JULY-SEPTEMBER 2007, pp. 430-440 and Kuo-ching Liang et al. in 40th Annual Conference on Information Sciences and Systems, IEEE, 2006, pp. 1599-1604 provide further mathematical methods for DNA sequence analysis using Hidden Markov Model.

SUMMARY

The present invention seeks to solve at least one of the problems existing in the related art to at least some extent.

According to a first aspect of the present invention, there is provided a method of determining base information of a predetermined region in a fetal genome according to claim 1. According to the invention, the method comprises the steps as defined in claim 1. A formation of offspring genome equals to a random recombination with parental generation's genome (i.e., an interchange of haplotype recombination, and a random combination of gametes). For pregnant plasma, if a fetal haplotype (a recombination of parental haplotypes) is assumed as hidden states, sequencing data of the plasma may be used as observations (observing sequence), transition probabilities, observation symbol probabilities and initial state distribution may be deduced in virtue of prior data, then the most possible fetal haplotype recombination may be determined using a hidden Markov Model based on Viterbi algorithm, so as to obtain more information of fetus prior to birth. Thus, according to embodiments of the present disclosure, in virtue of the hidden Markov Model, for example using the Viterbi algorithm, and referring to genetic information of a related individual, nucleic acid sequence of a predetermined region in a fetal genome may be determined, by which a prenatal genetic detection may be effectively performed with genetic information of fetal genome.

According to a second aspect of the present invention, there is provided a system for determining base information of a predetermined region in a fetal genome according to claim 9. According to the present invention, the system comprises the steps as defined in claim 9. Using the system may effectively implement the above method of determining base information of a predetermined region in a fetal genome, which determines nucleic acid sequence of a predetermined region in a fetal genome may be determined in virtue of the hidden Markov Model, for example using the Viterbi algorithm, and referring to genetic information of a related individual, by which a prenatal genetic detection may be effectively performed with genetic information of the fetal genome.

According to a third aspect of the present invention, there is provided a computer readable medium according to claim 14. According to the present invention, the computer readable medium including a plurality of instructions is adapted for determining base information of a predetermined region based on a sequencing result of a fetus combining with genetic information of a related individual using a hidden Markov Model which causes an apparatus to perform the steps as defined in claim 14. Using the computer readable medium of the present disclosure may effectively execute the plurality of instructions by a processor, to determine a nucleic acid sequence of the predetermined region in the fetal genome in virtue of the hidden Markov Model, for example using the Viterbi algorithm based on the sequencing data of the fetus combining with genetic information of a related individual, by which prenatal genetic detection may be effectively performed with genetic information of the fetal genome.

Additional aspects and advantages of embodiments of present disclosure will be given in part in the following descriptions, become apparent in part from the following descriptions, or be learned from the practice of the embodiments of the present disclosure.

BRIEF DESCRIPTION OF THE DRAWINGS

These and other aspects and advantages of embodiments of the present disclosure will become apparent and more readily appreciated from the following descriptions made with reference the accompanying drawings, in which:

Fig.1 is a flow chart showing an analyzing process using a hidden Markov Model according to an embodiment of the present disclosure; and
Fig.2 is a schematic diagram showing a system for determining base information of a predetermined region in a fetal genome according to an embodiment of the present disclosure.

DETAILED DESCRIPTION

Reference will be made in detail to embodiments of the present disclosure. The same or similar elements and the elements having same or similar functions are denoted by like reference numerals throughout the descriptions. The embodiments described herein with reference to drawings are explanatory, illustrative, and used to generally understand the present disclosure. The embodiments shall not be construed to limit the present disclosure.

It should note that terms such as "first" and "second" are used herein for purposes of description and are not intended to indicate or imply relative importance or significance. Thus, features defined with "first", "second" may explicitly or implicitly include one or more the features. Furthermore, in the description of the present disclosure, unless otherwise stated, "a/the plurality of" means two or more.

**Method of determining base information of a predetermined region in a fetal genome**

In a first aspect of the present disclosure, there is provided a method of determining base information of a predetermined region in a fetal genome. According to embodiments of the present disclosure, the method may comprise:

firstly, extracting a genomic DNA sample of a fetus from pregnant peripheral blood, constructing a sequencing library based on a genomic DNA sample of a fetus. According to the present disclosure, the source of the genomic DNA sample of the fetus is peripheral blood from a pregnant woman. Using the pregnant peripheral blood as the source of the genomic DNA sample of the fetus may effectively realize obtaining the genomic DNA sample of the fetus by noninvasive sampling, by which the fetal genome may be effectively monitored in the premise of having no influence on normal development of fetal growth. As for methods and processes of constructing a sequencing library for the nucleic acid sample, a person skilled in the art may appropriately select depending on different sequencing technology. Detailed process may refer to procedure provided by sequencer manufacturer, such as Illumina Company, for example, refer to Multiplexing Sample Preparation Guide (Part#1005361; Feb 2010) or Paired-End SamplePrep Guide (Part#1005063; Feb 2010) from Illumina Company, which are incorporated herein for reference. According to embodiments of the present disclosure, methods and devices for extracting a nucleic acid from a biological sample are not subjected to any special restrictions, which may be performed using a commercial nucleic acid extracting kit.

After being constructed, obtained sequencing library is applied to a sequencer, to obtain a corresponding sequencing result consisting of a plurality of sequencing data. According to embodiments of the present disclosure, methods and devices for sequencing are not subjected to any special restrictions, including but not limited to Chain Termination Method (Sanger); a high-throughput sequencing method is preferred. Thus, using characteristics being high-throughput and deep sequencing of these apparatus, efficiency may be further improved, by which precise and accuracy of subsequent analysis with sequencing data, such as statistical test, may be further improved. The high-throughput sequencing method includes but not limited to a Next-Generation sequencing technology or a single sequencing technology. The Next-Generation sequencing platform (Metzker ML. Sequencing technologies-the next generation. Nat Rev Genet. 2010 Jan; 11(1):31-46) includes but not limited to Illumina-Solexa (GATM, HiSeq2000TM, etc), ABI-Solid and Roche-454 (pyrosequencing) sequencing platform; the single sequencing platform (technology) includes but not limited to True Single Molecule DNA sequencing of Helicos Company, single molecule real-time (SMRT™) of Pacific Biosciences Company, and nonapore sequencing technology of Oxford Nanopore Technologies (Rusk, Nicole (2009-04-01). Cheap Third-Generation Sequencing. Nature Methods 6 (4): 244-245), etc. With gradual development of sequencing technology, a person skilled in the art may understand other sequencing methods and apparatuses may also be used for whole genome sequencing. According to specific examples of the present disclosure, the whole genome sequencing library may be subjected to sequencing by at least one selected from Illumina-Solexa, ABI-SOLiD, Roche-454 and a single molecule sequencing apparatus.

Optionally, after being obtained, the sequencing result may be aligned to a reference sequence, to determine sequencing data corresponding to the predetermined region. Term of "predetermined region" used herein should be broadly understood, referring to any region of a nucleic acid molecule containing a possible predetermined event. For SNP analysis, it may be a region containing SNP site. For analyzing chromosome aneuploidy, the predetermined region refers to entire or part of the chromosome to be analyzed, i.e., selecting sequencing data deriving from the chromosome. Methods of selecting sequencing data deriving from a corresponding region in the sequencing result are not subjected to any special restrictions. According to embodiments of the present disclosure, all obtained sequencing data may be aligned to a reference sequence with a known nucleic acid, to obtain the sequencing data deriving from the predetermined region. In addition, according to embodiments of the present disclosure, the predetermined region may also be a plurality of dispersal points which are not discontinuous in a genome. According to embodiments of the present disclosure, a type of used reference sequence may be not subjected to any special restrictions, which may be any known sequences contained a target region. According to embodiments of the present disclosure, the reference sequence may use a known human reference genome. For example, according to embodiments of the present disclosure, the human reference genome is NCBI 36.3, HG18. In addition, according to embodiments of the present disclosure, alignment methods are not subjected to any special restrictions. According to specific examples, SOAP may be used for alignment.

Then, determining a part of a nucleic acid sequence of the predetermined region based on sequencing data corresponding to the predetermined region; and determining other parts of the nucleic acid sequence based on determined part of the nucleic acid sequence of the predetermined region using Viterbi algorithm, to obtain the nucleic acid sequence of the predetermined region. According to the present invention, the base information of the predetermined region is determined based on the sequencing result of the fetus combining with genetic information of a related individual using a hidden Markov Model. According to embodiments of the present disclosure, the base information of the predetermined region is determined using the hidden Markov Model is performed based on Viterbi algorithm. Thus, a prenatal genetic detection may be effectively performed with genetic information of fetal genome.

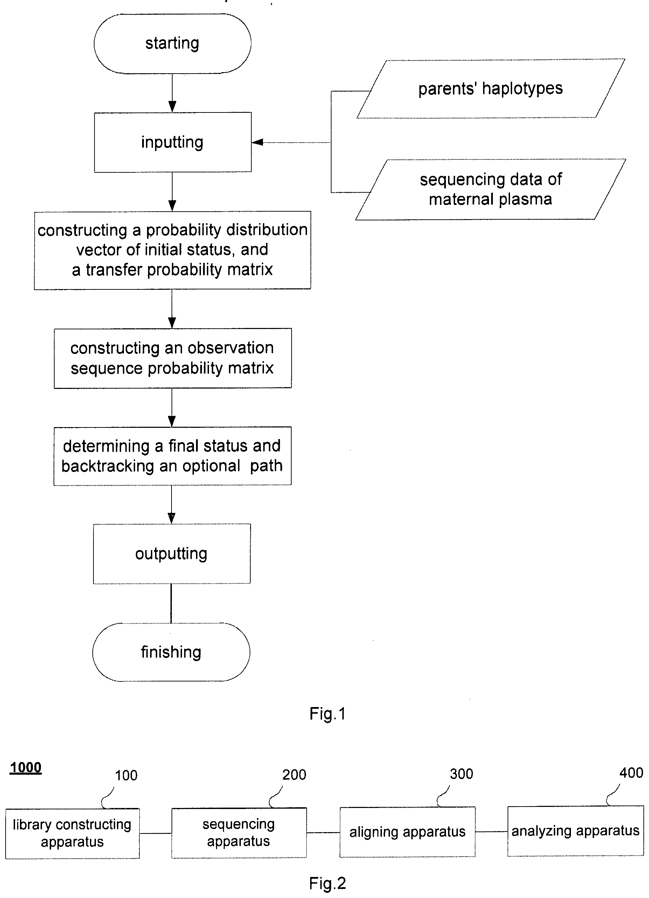

Referring to Fig.1, a principal for analysis using Viterbi algorithm in virtue of a hidden Markov Model is described in details below:

In the genetic sense, term of "a related individual" refers to individuals having a genetic relationship with a fetus. For example, according to embodiments of the present disclosure, "a related individual" may be a rental generation of a fetus, such as parents. Thus, a formation of offspring genome equals to a random recombination with parental generation's genome (i.e., an interchange of haplotype recombination, and a random combination of gametes). For pregnant plasma, if a fetal haplotype (a recombination of parental haplotypes) is assumed as hidden states, sequencing data of the plasma may be used as observations (observing sequence), transition probabilities, observation symbol probabilities and initial state distribution may be deduced in virtue of prior data, then the most possible fetal haplotype recombination may be determined using a hidden Markov Model based on Viterbi algorithm, so as to obtain more information of fetus prior to birth.

Steps of analyzing are show below in details:

Marker:

I. the number of sites to be detected is N.

II. haplotypes of parents are respectively recorded as $FH = \{fh_0, fh_1\}$ and $MH = \{mh_0, mh_1\}$, in which

$$mh_k = \{m_{1,k},...,m_{i,k},...,m_{N,k}\}, \quad fh_k = \{f_{1,k},...,f_{i,k},...,f_{N,k}\},$$

$$\forall fh_{i,k}, mh_{i,k} \in \{A, C, G, T\},$$

$$k \in \{0,1\}, \quad i = 1, 2, 3, ..., N.$$

III. Unknown fetal haplotype is recorded as $H = \{h_0, h_1\}$, particularly, $h_0$ and $h_1$ respectively represent inheriting from mother and father.

$$h_0 = \{m_{1,x_1},...,m_{i,x_i},...,m_{N,x_N}\}, \quad h_1 = \{f_{1,y_1},...,f_{i,y_i},...,f_{N,y_N}\},$$

in which $x_i \in \{0,1\}$, $y_i \in \{0,1\}$,

Subscripts $x_i$ and $y_i$ respectively present sequence pairs, and $q_i = \{x_i, y_i\}$ represents the hidden states which need to be decoded.

While, all hidden states possible presenting constitutes a set $Q$.

IV. Sequencing data is recorded as $S = \{s_1,..., s_i,..., s_N\}$

in which $s_i = \{n_{i,A}, n_{i,C}, n_{i,G}, n_{i,G}\}$ represents sequencing information of a site, containing the number of four bases, A, C, T and G.

V. A mean fetal concentration and a mean sequencing error rate are respectively recorded as $\varepsilon$ and $e$.

Step 1, constructing a probability distribution vector of an initial state and a transition matrix of haplotypes recombination:

I. The probability distribution of the initial states is recorded as $\pi = \{\pi_j\}$ ($j \in Q$).

According to embodiments of the present disclosure, under a circumstance of having no reference data, it may

assume that $\pi_j = \Pr(q_1 = j) \triangleq \dfrac{1}{4}$, i.e., possibilities of each hidden state presenting at the first site are equal.

II. According to embodiments of the present disclosure, a probability of haplotype recombination is recorded as $p_r = re/N$, in which re represents a mean times of human gamete recombinations, with a prior data ranging from 25 to 30.

III. According to embodiments of the present disclosure, a transition matrix of haplotypes recombination is recorded as $A = \{a_{jk}\}$ ($j,k \in Q$), in which $a_{jk}$ represents a probability of hidden states transition, i.e.,

$$a_{jk} = \Pr(q_i = k | q_{i-1} = j) = \begin{cases} (1 - p_r)^2 & x_i = x_{i-1}, y_i = y_{i-1} \\ (1 - p_r) \cdot p_r & x_i = x_{i-1}, y_i \neq y_{i-1} \quad or \quad x_i \neq x_{i-1}, y_i = y_{i-1} \\ p_r^2 & x_i \neq x_{i-1}, y_i \neq y_{i-1} \end{cases},$$

Subscripts $x_i$ and $y_i$ of fetal haplotypes $h_0 = \{m_{1,x_1},..., m_{i,x_i}..., m_{N,x_N}\}$ and $h_1 = \{f_{1,y_1},..., f_{i,y_i}..., f_{N,y_N}\}$ constitute a sequence pair, $q_i = \{x_i, y_i\}$ constitute the hidden states to be encoded. For example, $x_i = 0$ represents "in a maternal chromosome, an allele in the corresponding locus is $m_{i,0}$".

Step 2, constructing a probability matrix of observations:

According to embodiments of the present disclosure, the probability matrix of observations is recorded as $B=\{b_{i,j}(s_i)\}$ ($i = 1,2,3,..., N, j \in Q$), in which $b_{i,j}(s_i)$ represents "an observed possibility of this sequencing information in a site i, considering maternal haplotype and fetal haplotype (state j, $j=\{x_i,\ y_i\}$)", i.e.,

$$b_{i,j}\left(s_i\right) = \Pr\left(s_i \mid q_i = j, \{m_0, m_1\}\right)$$
$$= \frac{(n_{i,A} + n_{i,C} + n_{i,G} + n_{i,T})!}{n_{i,A}! n_{i,C}! n_{i,G}! n_{i,T}!} \cdot \left(P_{i,A}\right)^{n_{i,A}} \cdot \left(P_{i,C}\right)^{n_{i,C}} \cdot \left(P_{i,G}\right)^{n_{i,G}} \cdot \left(P_{i,T}\right)^{n_{i,T}},$$

in which $P_{i,base}$ represents "a possibility of a base in a site i, considering maternal haplotype and fetal haplotype (state j, $j=\{x_i,\ y_i\}$)", i.e.,

$$P_{i,base} = \Pr\left(base \mid q_i = j, \{m_0, m_1\}\right)$$
$$= \sum_{k \in \{0,1\}} \frac{1}{2}(1 - \varepsilon)\Delta\left(base, m_k\right) + \frac{1}{2}\varepsilon \cdot \Delta\left(base, m_{x_i}\right) + \frac{1}{2}\varepsilon \cdot \Delta\left(base, f_{y_i}\right),$$

in which, an indicator function is $\Delta\left(x,y\right) = \begin{cases} 1 - e & x = y \\ e/3 & x \neq y \end{cases}$.

Such step is to perform HMM parameter, calculating a probability distribution of observation in each site $b_{i,j}(s_i)$, i.e., calculating a possibility presenting current sequencing data (observations) in the pregnant plasma, assuming different fetal haplotypes in each site.

Step 3, constructing a partial probability matrix, and a reversal cursor (taking an example of constructing a one-dimensional probability matrix):

Definition: partial probability $\delta_i\left(q_i\right) = \left(\max_{q_{i-1} \in Q} \delta_i\left(q_i\right) \cdot a_{q_{i-1} q_i}\right) \cdot b_{i, q_i}\left(s_i\right)$,

Definition: reversal cursor $\Psi_i\left(q_i\right) = \arg\max_{q_{i-1} \in Q} \delta_i\left(q_i\right) \cdot a_{q_{i-1} q_i}$.

Terms of "partial probability $\delta_i(q_i)$" and "reversal cursor $\Psi_i(q_i)$" used herein both follow classic definitions of Viterbi algorithm. Detailed descriptions for the definition of the parameter may refer to Lawrence R. Rabiner, PROCEEDINGS OF THE IEEE, Vol.77, No.2, February 1989, which is incorporated herein by reference.

Step 4, determining a final state, and tracing back an optional path

Determination of the final state, $q_N^* = \arg\max_{q_N \in Q} \delta_N\left(q_N\right)$.

The most possible fetal haplotype $q_i^* = \Psi_i\left(q_i\right)$ ($i = 1,2,3,..., N - 1$) is obtained by tracing back the optional path based on the reversal curse.

Step 5, outputting a result

Thus, the sequence of the fetal genome may be effectively analyzed. Comparing to other existing method of antenatal detection, the method of the present disclosure may have following technical advantages, mainly embodying in accuracy and amount of genetic information obtainable:

1) According to embodiments of the present disclosure, a site to be detected is not limited to a parental site, for a maternal site, i.e., a maternal heterozygous site, whether a fetus inherits a maternal pathopoiesia site may also be detected excellently, with an accuracy up to 95% or more; and a plurality of abnormality types can be detected, which enlarges a range of disease detection.

2) According to embodiments of the present disclosure, information of a plurality of site and diseases may be obtained by one time of sequencing; while those gene sequence, having a low coverage in the pregnant plasma which is not able to be accurately determined only by enhancing sequencing depth, may be obtained by the method of the present disclosure, with an accurate and liable result.

3. According to embodiments of the present disclosure, a plotting with a genetic disease may be performed, some related diseases may be directly deduced with information of other sites, with a large amount of information obtained for one time, which has a more instructive meaning for clinical detection.

In addition, according to embodiments of the present disclosure, the method of determining base information of a predetermined region in a fetal genome, not limited to a certain genetic polymorphic sites such as SNP or STR, is adapted for all genetic polymorphic sites, which may be parallel used for a plurality of sites, to verify each other. Besides applying to antenatal noninvasive detect genomic information of a fetus, achieving a purpose of disease detection, the method of the present disclosure may also be used in noninvasive antenatal paternity identification, i.e., determining an identity of a fetus' father prior birth, providing assistance for disputes involving rearing responsibilities and obligations, property and sexual assault cases, etc.

**System for determining base information of a predetermined region in a fetal genome**

In another aspect of the present disclosure, there is provided a system for determining base information of a predetermined region in a fetal genome. According to embodiments of the present disclosure, referring to Fig.2, the system 1000 may comprises: a library constructing apparatus 100, a sequencing apparatus 200 and an analyzing apparatus 400.

According to embodiments of the present disclosure, the library constructing apparatus 100 is adapted for constructing sequencing library based on a genomic DNA sample of a fetus. According to embodiments of the present disclosure, the sequencing apparatus 200 is connected to the library constructing apparatus 100, and adapted for subjecting the sequencing library to sequencing, to obtain a sequencing result of the fetus consisting of a plurality of sequencing data. According to embodiments of the present disclosure, the system 1000 may also comprise a DNA sample extracting apparatus, adapted for extracting the genomic DNA sample of the fetus from pregnant peripheral blood. Thus, the system may be adapted for noninvasive antenatal detection.

According to embodiments of the present disclosure, optionally, the system may also comprise an aligning apparatus 300. According to embodiments of the present disclosure, the aligning apparatus 300 is connected to the sequencing apparatus 200, and adapted for aligning the sequencing result of the fetus to a reference sequence, to determine sequencing result deriving from the predetermined region. According to embodiments of the present disclosure, methods and devices for sequencing are not subjected to any special restrictions, including but not limited to Chain Termination Method (Sanger); a high-throughput sequencing method is preferred. Thus, using characteristics being high-throughput and deep sequencing of these apparatus, efficiency may be further improved, by which precise and accuracy of subsequent analysis with sequencing data, such as statistical test, may be further improved. The high-throughput sequencing method includes but not limited to a Next-Generation sequencing technology or a single sequencing technology. The Next-Generation sequencing platform (Metzker ML. Sequencing technologies-the next generation. Nat Rev Genet. 2010 Jan; 11(1):31-46) includes but not limited to Illumina-Solexa (GATM, HiSeq2000TM, etc), ABI-Solid and Roche-454 (pyrosequencing) sequencing platform; the single sequencing platform (technology) includes but not limited to True Single Molecule DNA sequencing of Helicos Company, single molecule real-time (SMRT™) of Pacific Biosciences Company, and nonapore sequencing technology of Oxford Nanopore Technologies (Rusk, Nicole (2009-04-01). Cheap Third-Generation Sequencing. Nature Methods 6 (4): 244-245), etc. With gradual development of sequencing technology, a person skilled in the art may understand other sequencing methods and apparatuses may also be used for whole genome sequencing. According to specific examples of the present disclosure, the whole genome sequencing library may be subjected to sequencing by at least one selected from Illumina-Solexa, ABI-SOLiD, Roche-454 and a single molecule sequencing apparatus. According to embodiments of the present disclosure, a type of used reference sequence may be not subjected to any special restrictions, which may be any known sequences contained a target region. According to embodiments of the present disclosure, the reference sequence may use a known human reference genome. For example, according to embodiments of the present disclosure, the human reference genome is NCBI 36.3, HG18. In addition, according to embodiments of the present disclosure, alignment methods are not subjected to any special restrictions. According to specific examples, SOAP may be used for alignment.

According to embodiments of the present disclosure, the analyzing apparatus 400 is connected to the sequencing apparatus, and adapted for determining the base information of the predetermined region based on the sequencing result of the fetus combining with genetic information of a related individual using a hidden Markov Model.

According to embodiments of the present disclosure, in the Viterbi algorithm, 0.25 is used as a probability distribution of an initial status, re/N is used as a recombination probability, with re being 25~30, preferably re being 25, and N being a length of the predetermined region,

$$a_{jk} = \mathrm{Pr}\left(q_i = k \middle| q_{i-1} = j\right) = \begin{cases} \left(1-p_r\right)^2 & x_i = x_{i-1}, y_i = y_{i-1} \\ \left(1-p_r\right) \cdot p_r & x_i = x_{i-1}, y_i \neq y_{i-1} \quad or \quad x_i \neq x_{i-1}, y_i = y_{i-1} \\ p_r^2 & x_i \neq x_{i-1}, y_i \neq y_{i-1} \end{cases} \quad \text{is}$$

used as a recombination transition matrix with $p_r$ being re/N.

According to embodiments of the present disclosure, the aligning apparatus is adapted for determining a base having the highest probability based on a formula of

$$P_{i,base} = \sum_{k\in\{0,1\}} \frac{1}{2}(1-\varepsilon)\Delta\left(base, m_k\right) + \frac{1}{2}\varepsilon \cdot \Delta\left(base, m_{x_i}\right) + \frac{1}{2}\varepsilon \cdot \Delta\left(base, f_{y_i}\right)$$

wherein $\Delta\left(x,y\right) = \begin{cases} 1-e & x=y \\ e/3 & x \neq y \end{cases}$ .

Analysis with sequencing data, which is detailed described above, is also adapted to the system for determining base information of a predetermined region in a fetal genome, which is omitted for brevity.

Thus, using the system may effectively implement the above method of determining base information of a predetermined region in a fetal genome, which may determine nucleic acid sequence of a predetermined region in a fetal genome may be determined in virtue of the hidden Markov Model, for example using the Viterbi algorithm, and referring to genetic information of a related individual, by which a prenatal genetic detection may be effectively performed with genetic information of the fetal genome.

In addition, according to embodiments of the present disclosure, the predetermined region is a site previously determined as having a genetic polymorphism, and the genetic polymorphism is at least one selected from single nucleotide polymorphism and STR.

Terms of "connected" should be broadly understood, which may refer to a direct connection or indirect connection, as long as achieving the above functional connection.

It should note that a person skilled in the art may understand that features and advantages of the method of determining base information of a predetermined region in a fetal genome described above may also adapted to the system for determining base information of a predetermined region in a fetal genome, which are omitted for brevity.

**Computer readable medium**

In a further aspect of the present disclosure, there is provided a computer readable medium. According to embodiments of the present disclosure, the computer readable medium includes a plurality of instructions, adapted for determining base information of a predetermined region based on a sequencing result of a fetus combining with genetic information of a related individual using a hidden Markov Model. Thus, using the computer readable medium may effectively implement the above method of determining base information of a predetermined region in a fetal genome, which may determine nucleic acid sequence of a predetermined region in a fetal genome may be determined in virtue of the hidden Markov Model, for example using the Viterbi algorithm, and referring to genetic information of a related individual, by which a prenatal genetic detection may be effectively performed with genetic information of the fetal genome.

According to embodiments of the present disclosure, the plurality of instructions are adapted for determining the base information of the predetermined region using the hidden Markov model based on Viterbi algorithm. According to embodiments of the present disclosure, in the Viterbi algorithm, 0.25 is used as a probability distribution of an initial status, re/N is used as a recombination probability, with re being 25~30, preferably re being 25, and N being a length of the predetermined region,

$$a_{jk} = \mathrm{Pr}\left(q_i = k \middle| q_{i-1} = j\right) = \begin{cases} \left(1-p_r\right)^2 & x_i = x_{i-1}, y_i = y_{i-1} \\ \left(1-p_r\right) \cdot p_r & x_i = x_{i-1}, y_i \neq y_{i-1} \quad or \quad x_i \neq x_{i-1}, y_i = y_{i-1} \\ p_r^2 & x_i \neq x_{i-1}, y_i \neq y_{i-1} \end{cases} \quad \text{is}$$

used as a recombination transition matrix with $p_r$ being re/N.

According to embodiments of the present disclosure, the plurality of instructions are further adapted for determining a base having the highest probability based on based on a formula of

$$P_{i,base} = \sum_{k \in \{0,1\}} \frac{1}{2}(1-\varepsilon)\Delta(base, m_k) + \frac{1}{2}\varepsilon \cdot \Delta(base, m_{x_i}) + \frac{1}{2}\varepsilon \cdot \Delta(base, f_{y_i})$$

wherein $\Delta(x,y) = \begin{cases} 1-e & x=y \\ e/3 & x \neq y \end{cases}$ .

Analysis with sequencing data, which is detailed described above, is also adapted to the computer readable medium, which is omitted for brevity.

In addition, according to embodiments of the present disclosure, the predetermined region is a site previously determined as having a genetic polymorphism, and the genetic polymorphism is at least one selected from single nucleotide polymorphism and STR.

As to the specification, "computer readable medium" may be any device adaptive for including, storing, communicating, propagating or transferring programs to be used by or in combination with the instruction execution system, device or equipment. More specific examples of the computer readable medium comprise but are not limited to: an electronic connection (an electronic device) with one or more wires, a portable computer enclosure (a magnetic device), a random access memory (RAM), a read only memory (ROM), an erasable programmable read-only memory (EPROM or a flash memory), an optical fiber device and a portable compact disk read-only memory (CDROM). In addition, the computer readable medium may even be a paper or other appropriate medium capable of printing programs thereon, this is because, for example, the paper or other appropriate medium may be optically scanned and then edited, decrypted or processed with other appropriate methods when necessary to obtain the programs in an electric manner, and then the programs may be stored in the computer memories.

It should be understood that each part of the present disclosure may be realized by the hardware, software, firmware or their combination. In the above embodiments, a plurality of steps or methods may be realized by the software or firmware stored in the memory and executed by the appropriate instruction execution system. For example, if it is realized by the hardware, likewise in another embodiment, the steps or methods may be realized by one or a combination of the following techniques known in the art: a discrete logic circuit having a logic gate circuit for realizing a logic function of a data signal, an application-specific integrated circuit having an appropriate combination logic gate circuit, a programmable gate array (PGA), a field programmable gate array (FPGA), etc.

Those skilled in the art shall understand that all or parts of the steps in the above exemplifying method of the present disclosure may be achieved by commanding the related hardware with programs. The programs may be stored in a computer readable storage medium, and the programs comprise one or a combination of the steps in the method embodiments of the present disclosure when run on a computer.

In addition, each function cell of the embodiments of the present disclosure may be integrated in a processing module, or these cells may be separate physical existence, or two or more cells are integrated in a processing module. The integrated module may be realized in a form of hardware or in a form of software function modules. When the integrated module is realized in a form of software function module and is sold or used as a standalone product, the integrated module may be stored in a computer readable storage medium.

Reference will be made in detail to examples of the present disclosure. It would be appreciated by those skilled in the art that the following examples are explanatory. If the specific technology or conditions are not specified in the examples, a step will be performed in accordance with the techniques or conditions described in the literature in the art (for example, referring to J. Sambrook, et al. (translated by Huang PT), Molecular Cloning: A Laboratory Manual, 3rd Ed., Science Press) or in accordance with the product instructions. If the manufacturers of reagents or instruments are not specified, the reagents or instruments may be commercially available, for example, from Illumina company.

General method

The method according to embodiments of the present disclosure mainly comprises following steps:

1) noninvasive sampling a pregnant sample containing fetal genetic materials, extracting genomic DNA therefrom;
2) extracting and purifying genomic DNA sample from family members of the fetus, such as parents or grandparents thereof;

3) constructing a sequencing library with every genetic material in accordance with an requirement for different sequencing platform;

4) filtering obtained sequencing data, with filtering criteria based on quality value, adaptor contamination and etc;

5) assembling obtained high-quality sequences as required, aligning an assembled result to a human genome reference sequence, to obtain uniquely-mapped sequences for analyzing using the model.

Analysis model

Marker:

I. the number of sites to be detected is N.

II. haplotypes of parents are respectively recorded as $FH = \{fh_0, fh_1\}$ and $MH = \{mh_0, mh_1\}$, in which

$$mh_k = \{m_{1,k},...,m_{i,k},...,m_{N,k}\}, \quad fh_k = \{f_{1,k},...,f_{i,k},...,f_{N,k}\},$$

$$\forall fh_{i,k}, mh_{i,k} \in \{A, C, G, T\},$$

$$k \in \{0,1\}, \quad i = 1,2,3,...,N.$$

III. Unknown fetal haplotype is recorded as $H = \{h_0, h_1\}$, particularly, $h_0$ and $h_1$ respectively represent inheriting from mother and father.

$$h_0 = \{m_{1,x_1},...,m_{i,x_i},...,m_{N,x_N}\}, \quad h_1 = \{f_{1,y_1},...,f_{i,y_i},...,f_{N,y_N}\},$$

in which $x_i \in \{0,1\}$, $y_i \in \{0,1\}$,

Subscripts $x_i$ and $y_i$ respectively present sequence pairs, and $q_i = \{x_i, y_i\}$ represents the hidden states which need to be decoded.

While, all hidden states possible presenting constitutes a set $Q$.

IV. Sequencing data is recorded as $S = \{s_1,..., s_i,..., s_N\}$

in which $s_i = \{n_{i,A}, n_{i,C}, n_{i,G}, n_{i,G}\}$ represents sequencing information of a site, containing the number of four bases, A, C, T and G.

V. A mean fetal concentration and a mean sequencing error rate are respectively recorded as $\varepsilon$ and $e$.

Step 1, constructing a probability distribution vector of an initial state and a transition matrix of haplotypes recombination:

I. The probability distribution of the initial states is recorded as $\pi = \{\pi_j\}$ ($j \in Q$). According to embodiments of the present disclosure, under a circumstance of having no reference data, it may assume that

$$\pi_j = \Pr(q_1 = j) \triangleq \frac{1}{4},$$ i.e., possibilities of each hidden state presenting at the first site are equal.

II. According to embodiments of the present disclosure, a probability of haplotype recombination is recorded as $p_r = re/N$, in which re represents a mean times of human gamete recombinations, with a prior data ranging from 25 to 30.

III. According to embodiments of the present disclosure, a transition matrix of haplotypes recombination is recorded as $A = \{a_{jk}\}$ ($j,k \in Q$), in which $a_{jk}$ represents a probability of hidden states transition, i.e.,

$$a_{jk} = \Pr\left(q_i = k \mid q_{i-1} = j\right) = \begin{cases} \left(1 - p_r\right)^2 & x_i = x_{i-1}, y_i = y_{i-1} \\ \left(1 - p_r\right) \cdot p_r & x_i = x_{i-1}, y_i \neq y_{i-1} \quad \text{or} \quad x_i \neq x_{i-1}, y_i = y_{i-1} \\ p_r^2 & x_i \neq x_{i-1}, y_i \neq y_{i-1} \end{cases},$$

Subscripts $x_i$ and $y_i$ of fetal haplotypes $h_0 = \{m_{1,x_1},..., m_{i,x_i},..., m_{N,x_N}\}$ and $h_1 = \{f_{1,y_1},..., f_{i,y_i},..., f_{N,y_N}\}$ constitute a sequence pair, $q_i = \{x_i, y_i\}$ constitute the hidden states to be encoded. For example, $x_i = 0$ represents "in a maternal chromosome, an allele in the corresponding locus is $m_{i,0}$".

Step 2, constructing a probability matrix of observations:

According to embodiments of the present disclosure, the probability matrix of observations is recorded as $B = \{b_{i,j}(s_i)\}$ ($i = 1,2,3,..., N, j \in Q$), in which $b_{i,j}(s_i)$ represents "an observed possibility of this sequencing information in a site i, considering maternal haplotype and fetal haplotype (state j, $j=\{x_i, y_i\}$)", i.e.,

$$b_{i,j}\left(s_i\right) = \Pr\left(s_i \mid q_i = j, \{m_0, m_1\}\right)$$
$$= \frac{(n_{i,A} + n_{i,C} + n_{i,G} + n_{i,T})!}{n_{i,A}! n_{i,C}! n_{i,G}! n_{i,T}!} \cdot \left(P_{i,A}\right)^{n_{i,A}} \cdot \left(P_{i,C}\right)^{n_{i,C}} \cdot \left(P_{i,G}\right)^{n_{i,G}} \cdot \left(P_{i,T}\right)^{n_{i,T}},$$

in which $P_{i,base}$ represents "a possibility of a base in a site i, considering maternal haplotype and fetal haplotype (state j, $j=\{x_i, y_i\}$)", i.e.,

$$P_{i,base} = \Pr\left(base \mid q_i = j, \{m_0, m_1\}\right)$$
$$= \sum_{k \in \{0,1\}} \frac{1}{2}(1 - \varepsilon)\Delta\left(base, m_k\right) + \frac{1}{2}\varepsilon \cdot \Delta\left(base, m_{x_i}\right) + \frac{1}{2}\varepsilon \cdot \Delta\left(base, f_{y_i}\right),$$

in which, an indicator function is

$$\Delta\left(x, y\right) = \begin{cases} 1 - e & x = y \\ e/3 & x \neq y \end{cases}.$$

Step 3, constructing a partial probability matrix, and a reversal cursor (taking an example of constructing a one-dimensional probability matrix):

Definition: partial probability $\delta_i\left(q_i\right) = \left(\max_{q_{i-1} \in Q} \delta_i\left(q_i\right) \cdot a_{q_{i-1}q_i}\right) \cdot b_{i,q_i}\left(s_i\right),$

Definition: reversal cursor $\Psi_i\left(q_i\right) = \arg\max_{q_{i-1} \in Q} \delta_i\left(q_i\right) \cdot a_{q_{i-1}q_i}.$

Step 4, determining a final state, and tracing back an optional path

Determination of the final state, $q_N^* = \arg\max_{q_N \in Q} \delta_N\left(q_N\right).$

The most possible fetal haplotype $q_i^* = \Psi_i\left(q_i\right)$ ($i = 1, 2, 3, ..., N - 1$) is obtained by tracing back the optional path based on the reversal curse.

Step 5, outputting a result

EXAMPLE 1

Sample collection and treatment

(1) collected sample included: peripheral blood extracted from a father and a pregnant mother within a family, and fetal umbilical cord blood after birth, all of which were collected in a tube containing EDTA for anticoagulation; saliva were collected from four grandparents using a Oragene® DNA saliva collection/DNA purification kit OG-250.

(2) extracted saliva DNA of the four grandparents were subjected to genotyping using Infinium® HD Human610-Quad BeadChip gene chip.

(3) the peripheral blood collected from the pregnant mother was centrifuged with 1600 g at 4°C for 10 min, to separate blood cells and plasma. Then obtained plasma was centrifuged with 16000 g at 4°C for 10 min, to further remove residual leukocytes, to obtain final plasma of the pregnant mother. Then genomic DNA was extracted from the final plasma of the pregnant mother using TIANamp Micro DNA Kit (TIANGEN), to obtain a genomic DNA mixture of mother and fetus thereof. Then maternal genomic DNA was extracted from removed residual leukocytes. Obtained plasma DNA were subjected to library construction based on requirement for HiSeq2000™ sequencer of Illumia® sequencer. Constructed libraries were subjected to a distribution test using Agilent®Bioanalyzer 2100 to meet a requirement for fragment ranges. Then two libraries were subjected to quantification using Q-PCR method. Qualified libraries were subjected to sequencing using Illumina® HiSeq2000™ sequencer, with a sequencing cycle of PE101 index (i.e., pair-end 101 bp index sequencing), in which parameter settings and operations were based on Illumina® specifications (obtained at http://www.illumina.com/support/documentation.ilmn)

(4) parental peripheral blood, leukocytes extracted from maternal peripheral blood and fetal umbilical cord blood were extracted with their respective genomic DNA using TIANamp Micro DNA Kit (TIANGEN).

Except for plasma DNA sample, all obtained DNA sample needed to be fragmented using Covaris™ to have a length of 500 bp. Obtained DNA fragments and plasma DNA sample were subjected to library construction based on the requirement for HiSeq2000™ sequencer of Illumia® sequencer, with a detailed procedure:

End-reparing reacting system:

| | |
|---|---|
| 10× T4 Polynucleotide kinase buffer | 10 μL |
| dNTPs (10 mM) | 4 μL |
| T4 DNA polymerase | 5 μL |
| Klenow fragments | 1 μL |
| T4 Polynucleotide kinase | 5 μL |
| DNA fragments | 30 μL |
| ddH$_2$O | up to 100 μL |

After reacting at 20°C for 30 min, PCR Purification Kit (QIAGEN) was used in recycling end-repaired products. Then the recycled end-repaired products were finally dissolved in 34μL of EB buffer.

A reacting system for adding base A at end:

| | |
|---|---|
| 10× Klenow buffer | 5 μL |
| dATP (1 mM) | 10 μL |
| Klenow (3'-5' exo⁻) | 3 μL |
| DNA | 32 μL |

After incubating at 37°C for 30 min, obtained products were purified by MinElute® PCR Purification Kit (QIAGEN) and dissolved in 12 μL of EB buffer, to obtain DNA samples added with base A at end.

Ligating adaptor reacting system:

| | |
|---|---|
| 2× Rapid DNA ligating buffer | 25 μL |
| PEI Adapter oligo-mix (20 μM) | 10 μL |
| T4 DNA ligase | 5 μL |
| DNA sample added with base A at end | 10 μL |

After reacting at 20°C for 15 min, PCR Purification Kit (QIAGEN) was used in recycling ligated products. The ligated products were finally dissolved in 32 μL of EB buffer.

PCR reacting system:

| Ligated product | 10 $\mu$L |
|---|---|
| Phusion DNA Polymerase Mix | 25 $\mu$L |
| PCR primer (10 pmol/$\mu$L) | 1 $\mu$L |
| Index N (10 pmol/$\mu$L) | 1 $\mu$L |
| UltraPureTM Water | 130 $\mu$L |

Reacting procedure was shown as below:

| 98°C | 30 s | |
|---|---|---|
| 98°C | 10 s | |
| 65°C | 30 s | } 10 cycles |
| 72°C | 30 s | |
| 72°C | 5 min | |
| 4°C | Hold | |

PCR Purification Kit (QIAGEN) was used in recycling PCR products, which were finally dissolved in 50 $\mu$L of EB buffer. Constructed libraries were subjected to a distribution test using Agilent®Bioanalyzer 2100 to meet a requirement for fragment ranges. Then two libraries were subjected to quantification using Q-PCR method. Qualified libraries were subjected to sequencing using Illumina® HiSeq2000™ sequencer, with a sequencing cycle of PE101 index (i.e., pair-end 101 bp index sequencing), in which parameter settings and operations were based on Illumina® specifications (obtained at http://www.illumina.com/support/documentation.ilmn)

(5) parental and maternal genomes sequencing genotyping

a. the sequencing data were aligned to a human reference genome (Hg19, NCBI 36.3) using SOAP2.
b. obtained data were subjected to consensus sequence (CNS) construction using SOAPsnp (thousands of planning data were used for Southern Han (CHS) pedigree data).
c. genotypes of a maker site were extracted.

(6) determination of parents' haplotypes

a. constructing a group genotype matrix containing ancestors' and parents' genotypes, i.e., extracting genotypes in the marker site of parents, ancestors and Southern Han pedigree.
b. deducing parents' haplotypes using BEAGLE.

(7) determination of fetal haplotype

a. aligning plasma sequencing data to a human reference genome ((Hg19, NCBI 36.3) using SOAP2;
b. constructing a probability vector of initial states, and a transition matrix of haplotypes recombination,
constructing the probability vector of initial states: taking a model of non-reference data, i.e., probabilities of every initial states were equal, being 0.25.
constructing the transition matrix of haplotypes recombination: conservatively, $re$= 25 (others were same as descriptions in "general method");
c. calculating sequencing information of each site, and constructing a probability matrix of observations (others were same as descriptions in "general method");
d. constructing a partial probability matrix, and a reversal curse (others were same as descriptions in "general method");
e. determining a final state, and tracing back an optional path; and
f. outputting.

According to genotyping results, the accuracy thereof were shown below:

| | | mother | | | | | | total | | |
|---|---|---|---|---|---|---|---|---|---|---|
| | | homozygosis | | | heterozygosis | | | | | |
| | | site number | accurate number | accuracy | site number | accurate number | accuracy | site number | accurate number | accuracy |
| father autosome | homozygosis | 199,552 | 199,552 | 100.00% | 66,238 | 63,968 | 96.57% | 265,790 | 263,520 | 99.15% |
| | heterozygosis | 65,409 | 64,735 | 98.97% | 41,849 | 39,944 | 95.45% | 107,258 | 104,679 | 97.60% |
| | | 264,961 | 264,287 | 99.75% | 108,087 | 103,912 | 96.14% | 373,048 | 368,199 | 98.70% |
| chromosome X | | 4,881 | 4,881 | 100.00% | 1,718 | 1,478 | 86.03% | 6,599 | 6,359 | 96.36% |

Industrial Applicability

The method of determining base information of a predetermined region in a fetal genome, the system for determining base information of a predetermined region in a fetal genome and a computer readable medium according to the present invention may be effectively applied in analyzing the nucleic acid sequence of the predetermined region in the fetal genome.

Reference throughout this specification to "an embodiment," "some embodiments", "one embodiment", "another example", "an example", "a specific examples", or "some examples", means that a particular feature, structure, material, or characteristic described in connection with the embodiment or example is included in at least one embodiment or example of the present disclosure. Thus, the appearances of the phrases such as "in some embodiments," "in one embodiment", "in an embodiment", "in another example, "in an example," "in a specific examples," or "in some examples," in various places throughout this specification are not necessarily referring to the same embodiment or example of the present disclosure. Furthermore, the particular features, structures, materials, or characteristics may be combined in any suitable manner in one or more embodiments or examples.

**Claims**

1. A method of determining base information of a predetermined region in a fetal genome, comprising the following steps:

   extracting a genomic DNA sample of a fetus from pregnant peripheral blood,
   constructing a sequencing library based on the genomic DNA sample of the fetus;
   subjecting the sequencing library to sequencing, to obtain a sequencing result of the fetus consisting of a plurality of sequencing data; and
   determining the base information of the predetermined region based on the sequencing result of the fetus combining with genetic information of a related individual using a hidden Markov Model,
   wherein the base information of the predetermined region comprises the most possible fetal haplotype recombination,
   and the base information of the predetermined region is obtained by:

      constructing a probability distribution vector of an initial state and a transition matrix of haplotypes recombination;
      constructing a probability matrix of observations;
      constructing a partial probability matrix, and a reversal cursor;
      determining a final state, and tracing back an optional path; and
      outputting a result to analyze the DNA sequence of the fetal genome.

2. The method of claim 1, wherein the sequencing library is subjected to sequencing by at least one selected from Illumina-Solexa, ABI-Solid, Roche-454 and a single molecule sequencing apparatus.

3. The method of claim 1, further comprising a step of aligning the sequencing result of the fetus to a reference sequence, to determine sequencing result deriving from the predetermined region,
   wherein the step of aligning the sequencing result of the fetal genome to the reference sequence to determine sequencing result deriving from the predetermined region further comprises:

   determining a base having the highest probability based on a formula of

$$P_{i,base} = \sum_{k \in \{0,1\}} \frac{1}{2}(1-\varepsilon)\,\Delta\big(base, m_k\big) + \frac{1}{2}\varepsilon \cdot \Delta\big(base, m_{x_i}\big) + \frac{1}{2}\varepsilon \cdot \Delta\big(base, f_{y_i}\big)$$

wherein $\Delta(x,y) = \begin{cases} 1-e & x=y \\ e/3 & x \neq y \end{cases}$

4. The method of claim 3, wherein the reference sequence is a human reference genome.

5. The method of claim 1, wherein the related individual is a parent of the fetus.

6. The method of claim 1, wherein the step of determining the base information of the predetermined region using the hidden Markov Model is performed based on Viterbi algorithm,
   and in the Viterbi algorithm, 0.25 is used as a probability distribution of an initial status, re/N is used as recombination probabilities, with re being 25~30, and N being a length of the predetermined region,

$$a_{jk} = \Pr\left(q_i = k \mid q_{i-1} = j\right) = \begin{cases} \left(1 - p_r\right)^2 & x_i = x_{i-1}, y_i = y_{i-1} \\ \left(1 - p_r\right) \cdot p_r & x_i = x_{i-1}, y_i \neq y_{i-1} \quad or \quad x_i \neq x_{i-1}, y_i = y_{i-1} \\ p_r^2 & x_i \neq x_{i-1}, y_i \neq y_{i-1} \end{cases}$$

is used as a recombination transition matrix with $p_r$ being re/N.

7. The method of claim 6, wherein re is 25.

8. The method of claim 1, wherein the predetermined region is a site previously determined as having a genetic polymorphism,
   wherein the genetic polymorphism is at least one selected from single nucleotide polymorphism and STR.

9. A system for determining base information of a predetermined region in a fetal genome, comprising:

   a DNA sample extracting apparatus, adapted for extracting a genomic DNA sample of a fetus from pregnant peripheral blood,
   a library constructing apparatus, adapted for constructing sequencing library based on the genomic DNA sample of the fetus;
   a sequencing apparatus, connected to the library constructing apparatus, and adapted for subjecting the sequencing library to sequencing, to obtain a sequencing result of the fetus consisting of a plurality of sequencing data; and
   an analyzing apparatus, connected to the sequencing apparatus, and adapted for determining the base information of the predetermined region based on the sequencing result of the fetus combining with genetic information of a related individual using a hidden Markov Model,
   wherein the base information of the predetermined region comprises the most possible fetal haplotype recombination,
   and the base information of the predetermined region is obtained by:

      constructing a probability distribution vector of an initial state and a transition matrix of haplotypes recombination;
      constructing a probability matrix of observations;
      constructing a partial probability matrix, and a reversal cursor;
      determining a final state, and tracing back an optional path; and
      outputting a result to analyze the DNA sequence of the fetal genome.

10. The system of claim 9, wherein the sequencing apparatus is at least one selected from Illumina-Solexa, ABI-Solid, Roche-454 and a single molecule sequencing apparatus.

11. The system of claim 9, further comprising an aligning apparatus, connected to the sequencing apparatus, and adapted for aligning the sequencing result of the fetus to a reference sequence, to determine sequencing result deriving from the predetermined region,
    wherein the aligning apparatus is adapted for determining a base having the highest probability based on a formula of

$$P_{i,base} = \sum_{k\in\{0,1\}} \frac{1}{2}(1-\varepsilon)\Delta\left(base, m_k\right) + \frac{1}{2}\varepsilon \cdot \Delta\left(base, m_{x_i}\right) + \frac{1}{2}\varepsilon \cdot \Delta\left(base, f_{y_i}\right)$$

wherein $\Delta\left(x, y\right) = \begin{cases} 1-e & x=y \\ e/3 & x \neq y \end{cases}$ .

12. The system of claim 9, wherein the analyzing apparatus is adapted for determining the base information of the predetermined region using a hidden Markov Model based on Viterbi algorithm, wherein in the Viterbi algorithm, 0.25 is used as a probability distribution of an initial status, re/N is used as recombination probabilities, with re being 25~30, and N being a length of the predetermined region,

$$a_{jk} = \Pr\left(q_i = k \mid q_{i-1} = j\right) = \begin{cases} (1-p_r)^2 & x_i = x_{i-1}, y_i = y_{i-1} \\ (1-p_r)\cdot p_r & x_i = x_{i-1}, y_i \neq y_{i-1} \quad or \quad x_i \neq x_{i-1}, y_i = y_{i-1} \\ p_r^2 & x_i \neq x_{i-1}, y_i \neq y_{i-1} \end{cases}$$

is used as a recombination transition matrix with pr being re/N.

13. The system of claim 12, wherein re is 25.

14. A computer readable medium comprising a plurality of instructions, adapted for determining base information of a predetermined region based on a sequencing result of a fetus combining with genetic information of a related individual using a hidden Markov Model which causes the apparatus to perform:

   extracting a genomic DNA sample of a fetus from pregnant peripheral blood,
   constructing a sequencing library based on the genomic DNA sample of the fetus;
   subjecting the sequencing library to sequencing, to obtain a sequencing result of the fetus consisting of a plurality of sequencing data; and
   determining the base information of the predetermined region based on the sequencing result of the fetus combining with genetic information of a related individual using the hidden Markov Model,
   wherein the base information of the predetermined region comprises the most possible fetal haplotype recombination,
   and the base information of the predetermined region is obtained by:

   constructing a probability distribution vector of an initial state and a transition matrix of haplotypes recombination;
   constructing a probability matrix of observations;
   constructing a partial probability matrix, and a reversal cursor;
   determining a final state, and tracing back an optional path; and
   outputting a result to analyze the DNA sequence of the fetal genome.

15. The computer readable medium of claim 14, wherein the plurality of instructions are adapted for determining the base information of the predetermined region using the hidden Markov model based on Viterbi algorithm, wherein in the Viterbi algorithm, 0.25 is used as a probability distribution of an initial status, re/N is used as recombination probabilities, with re being 25~30, and N being a length of the predetermined region,

$$a_{jk} = \Pr\left(q_i = k \middle| q_{i-1} = j\right) = \begin{cases} \left(1-p_r\right)^2 & x_i = x_{i-1}, y_i = y_{i-1} \\ \left(1-p_r\right)\cdot p_r & x_i = x_{i-1}, y_i \neq y_{i-1} \quad or \quad x_i \neq x_{i-1}, y_i = y_{i-1} \\ p_r^2 & x_i \neq x_{i-1}, y_i \neq y_{i-1} \end{cases}$$

is used as a recombination transition matrix with $p_r$ being re/N.

16. The computer readable medium of claim 15, wherein re is 25.

17. The computer readable medium of claim 14, wherein the plurality of instructions are adapted for aligning the sequencing result of the fetus to a reference sequence, to determine sequencing result deriving from the predetermined region,
wherein the plurality of instructions are further adapted for determining a base having the highest probability based on a formula

$$P_{i,base} = \sum_{k \in \{0,1\}} \frac{1}{2}(1-\varepsilon)\Delta\left(base, m_k\right) + \frac{1}{2}\varepsilon \cdot \Delta\left(base, m_{x_i}\right) + \frac{1}{2}\varepsilon \cdot \Delta\left(base, f_{y_i}\right)$$

wherein $\Delta(x,y) = \begin{cases} 1-e & x = y \\ e/3 & x \neq y \end{cases}$ .

## Patentansprüche

1. Verfahren zum Bestimmen von einer Baseninformation von einer vorbestimmten Region in einem fötalem Genom, umfassend die folgenden Schritte:

Extrahieren von einer genomischen DNS-Probe von einem Fötus aus peripherem Schwangerenblut,
Konstruieren von einer Sequenzierungsbibliothek auf Basis von der genomischen DNS-Probe des Fötus;
Unterziehen von der Sequenzierungsbibliothek einer Sequenzierung, um ein Sequenzierungsergebnis von dem Fötus zu erhalten, das aus einer Vielzahl von Sequenzierungsdaten besteht; und
Bestimmen von der Baseninformation von der vorbestimmten Region auf Basis von dem Kombinieren von dem Sequenzierungsergebnis des Fötus mit genetischer Information von einem verwandten Individuum, wobei ein Hidden-Markov-Model verwendet wird,
wobei die Baseninformation von der vorbestimmten Region die maximal mögliche fötale Haplotyprekombination umfasst,
und die Baseninformation von der vorbestimmten Region erhalten wird durch:

das Konstruieren von einem Wahrscheinlichkeitsverteilungsvektor von einem Anfangszustand und einer Übergangsmatrix der Haplotypenrekombination;
das Konstruieren von einer Wahrscheinlichkeitsmatrix von Beobachtungen;
das Konstruieren von einer partiellen Wahrscheinlichkeitsmatrix und einem Reverscursor (Englisch: reversal cursor);
das Bestimmen von einem Endzustand und das Zurückverfolgen von einem optionalen Weg; und
das Ausgeben von einem Ergebnis, um die DNS-Sequenz von dem fötalen Genom zu analysieren.

2. Verfahren nach Anspruch 1, wobei die Sequenzierungsbibliothek einer Sequenzierung durch mindestens einen Apparat unterzogen wird, ausgewählt aus Illumina-Solexa-, ABI-Solid-, Roche-454- und einem Einzelmolekülsequenzierungsapparat.

3. Verfahren nach Anspruch 1, ferner umfassend einen Schritt des Abgleichens von dem Sequenzierungsergebnis von dem Fötus mit einer Bezugssequenz, um das Sequenzierungsergebnis, das sich von der vorbestimmten Region ableitet, zu bestimmen,
wobei der Schritt des Abgleichs von dem Sequenzierungsergebnis von dem fötalen Genom mit der Bezugssequenz, um das Sequenzierungsergebnis, das sich von der vorbestimmten Region ableitet, zu bestimmen, ferner das Folgende umfasst:

Bestimmen von einer Base, welche die höchste Wahrscheinlichkeit aufweist, und zwar basierend auf einer Formel

$$P_{i,base} = \sum_{k\in\{0,1\}} \frac{1}{2}(1-\varepsilon)\Delta\left(base,m_k\right) + \frac{1}{2}\varepsilon\cdot\Delta\left(base,m_{x_i}\right) + \frac{1}{2}\varepsilon\cdot\Delta\left(base,f_{y_i}\right)$$

wobei $\Delta(x,y) = \begin{cases} 1-e & x=y \\ e/3 & x\neq y \end{cases}$ .

4. Verfahren nach Anspruch 3, wobei die Bezugssequenz ein humanes Bezugsgenom ist.

5. Verfahren nach Anspruch 1, wobei das verwandte Individuum ein Elternteil des Fötus ist.

6. Verfahren nach Anspruch 1, wobei der Schritt des Bestimmens von der Baseninformation von der vorbestimmten Region durchgeführt wird, wobei das Hidden-Markov-Model auf Basis eines Viterbi-Algorithmus verwendet wird, und in dem Viterbi-Algorithmus 0,25 als eine Wahrscheinlichkeitsverteilung von einem Anfangsstatus verwendet wird, re/N als Rekombinationswahrscheinlichkeiten verwendet wird, wobei re 25~30 und N eine Länge von der vorbestimmten Region ist, und

$$a_{jk} = \Pr\left(q_i=k\middle|q_{i-1}=j\right) = \begin{cases} \left(1-p_r\right)^2 & x_i=x_{i-1}, y_i=y_{i-1} \\ \left(1-p_r\right)\cdot p_r & x_i=x_{i-1}, y_i\neq y_{i-1} \quad or \quad x_i\neq x_{i-1}, y_i=y_{i-1} \\ p_r^2 & x_i\neq x_{i-1}, y_i\neq y_{i-1} \end{cases}$$

als eine Rekombinationsübergangsmatrix verwendet wird, wobei $p_r$ re/N ist.

7. Verfahren nach Anspruch 6, wobei re 25 ist.

8. Verfahren nach Anspruch 1, wobei die vorbestimmte Region ein Ort ist, der zuvor als ein Ort bestimmt wurde, der einen genetischen Polymorphismus aufweist,
wobei der genetische Polymorphismus mindestens einer ausgewählt aus Einzelnukleotidpolymorphismus und STR ist.

9. System zum Bestimmen von einer Baseninformation von einer vorbestimmten Region in einem fötalem Genom, umfassend:

einen DNS-Proben-Extrahierapparat, der geeignet ist, um eine genomische DNS-Probe von einem Fötus aus peripherem Schwangerenblut zu extrahieren;
einen Bibliothekskonstruierapparat, der geeignet ist, um eine Sequenzierungsbibliothek auf Basis von der genomischen DNS-Probe des Fötus zu konstruieren;
einen Sequenzierungsapparat, der mit dem Bibliothekskonstruierapparat verbunden und geeignet ist, um die Sequenzierungsbibliothek einer Sequenzierung zu unterziehen, um ein Sequenzierungsergebnis von dem Fötus zu erhalten, das aus einer Vielzahl von Sequenzierungsdaten besteht; und

einen Analyseapparat, der mit dem Sequenzierungsapparat verbunden und geeignet ist, um die Baseninformation von der vorbestimmten Region auf Basis von dem Kombinieren von dem Sequenzierungsergebnis des Fötus mit genetischer Information von einem verwandten Individuum zu bestimmen, wobei ein Hidden-Markov-Model verwendet wird,

wobei die Baseninformation von der vorbestimmten Region die maximal mögliche fötale Haplotyprekombination umfasst,

und die Baseninformation von der vorbestimmten Region erhalten wird durch:

das Konstruieren von einem Wahrscheinlichkeitsverteilungsvektor von einem Anfangszustand und einer Übergangsmatrix der Haplotypenrekombination;
das Konstruieren von einer Wahrscheinlichkeitsmatrix von Beobachtungen;
das Konstruieren von einer partiellen Wahrscheinlichkeitsmatrix und einem Reverscursor (Englisch: reversal cursor);
das Bestimmen von einem Endzustand und das Zurückverfolgen von einem optionalen Weg; und
das Ausgeben von einem Ergebnis, um die DNS-Sequenz von dem fötalen Genom zu analysieren.

**10.** System nach Anspruch 9, wobei der Sequenzierungsapparat mindestens einer ist, ausgewählt aus einem Illumina-Solexa-, ABI-Solid-, Roche-454- und einem Einzelmolekülsequenzierungsapparat.

**11.** System nach Anspruch 9, ferner umfassend einen Abgleichapparat, der mit dem Sequenzierungsapparat verbunden und geeignet ist, das Sequenzierungsergebnis von dem Fötus mit einer Bezugssequenz abzugleichen, um das Sequenzierungsergebnis, das sich von der vorbestimmten Region ableitet, zu bestimmen, wobei der Abgleichapparat geeignet ist, um eine Base zu bestimmen, welche die höchste Wahrscheinlichkeit aufweist, und zwar basierend auf einer Formel

$$P_{i,base} = \sum_{k \in \{0,1\}} \frac{1}{2}(1-\varepsilon)\Delta\left(base, m_k\right) + \frac{1}{2}\varepsilon \cdot \Delta\left(base, m_{x_i}\right) + \frac{1}{2}\varepsilon \cdot \Delta\left(base, f_{y_i}\right)$$

wobei $\Delta(x,y) = \begin{cases} 1-e & x=y \\ e/3 & x \neq y \end{cases}$ .

**12.** System nach Anspruch 9, wobei der Analyseapparat geeignet ist, um die Baseninformation von der vorbestimmten Region zu bestimmen, wobei ein Hidden-Markov-Model auf Basis eines Viterbi-Algorithmus verwendet wird, wobei in dem Viterbi-Algorithmus 0,25 als eine Wahrscheinlichkeitsverteilung von einem Anfangsstatus verwendet wird, re/N als Rekombinationswahrscheinlichkeiten verwendet wird, wobei re 25~30 und N eine Länge von der vorbestimmten Region ist, und

$$a_{jk} = \Pr\left(q_i = k | q_{i-1} = j\right) = \begin{cases} \left(1-p_r\right)^2 & x_i = x_{i-1}, y_i = y_{i-1} \\ \left(1-p_r\right) \cdot p_r & x_i = x_{i-1}, y_i \neq y_{i-1} \quad or \quad x_i \neq x_{i-1}, y_i = y_{i-1} \\ p_r^2 & x_i \neq x_{i-1}, y_i \neq y_{i-1} \end{cases}$$

als eine Rekombinationsübergangsmatrix verwendet wird, wobei pr re/N ist.

**13.** System nach Anspruch 12, wobei re 25 ist.

**14.** Computerlesbares Medium, umfassend eine Vielzahl von Instruktionen, die geeignet sind, um die Baseninformation von einer vorbestimmten Region auf Basis von dem Kombinieren von einem Sequenzierungsergebnis des Fötus mit genetischer Information von einem verwandten Individuum zu bestimmen, wobei ein Hidden-Markov-Model verwendet wird, wodurch der Apparat veranlasst wird, das Folgende durchzuführen:

Extrahieren von einer genomischen DNS-Probe von einem Fötus aus peripherem Schwangerenblut,
Konstruieren von einer Sequenzierungsbibliothek auf Basis von der genomischen DNS-Probe des Fötus;
Unterziehen von der Sequenzierungsbibliothek einer Sequenzierung, um ein Sequenzierungsergebnis von dem Fötus zu erhalten, das aus einer Vielzahl von Sequenzierungsdaten besteht; und
Bestimmen von der Baseninformation von der vorbestimmten Region auf Basis von dem Kombinieren von dem Sequenzierungsergebnis des Fötus mit genetischer Information von einem verwandten Individuum, wobei das Hidden-Markov-Model verwendet wird,
wobei die Baseninformation von der vorbestimmten Region die maximal mögliche fötale Haplotyprekombination umfasst,
und die Baseninformation von der vorbestimmten Region erhalten wird durch:

das Konstruieren von einem Wahrscheinlichkeitsverteilungsvektor von einem Anfangszustand und einer Übergangsmatrix der Haplotypenrekombination;
das Konstruieren von einer Wahrscheinlichkeitsmatrix von Beobachtungen;
das Konstruieren von einer partiellen Wahrscheinlichkeitsmatrix und einem Reverscursor (Englisch: reversal cursor);
das Bestimmen von einem Endzustand und das Zurückverfolgen von einem optionalen Weg; und
das Ausgeben von einem Ergebnis, um die DNS-Sequenz von dem fötalen Genom zu analysieren.

15. Computerlesbares Medium nach Anspruch 14, wobei die Vielzahl von Instruktionen geeignet ist, um die Baseninformation von der vorbestimmten Region zu bestimmen, wobei das Hidden-Markov-Model auf Basis eines Viterbi-Algorithmus verwendet wird,
wobei in dem Viterbi-Algorithmus 0,25 als eine Wahrscheinlichkeitsverteilung von einem Anfangsstatus verwendet wird, re/N als Rekombinationswahrscheinlichkeiten verwendet wird, wobei re 25~30 und N eine Länge von der vorbestimmten Region ist, und

$$a_{jk} = \Pr\left(q_i = k \middle| q_{i-1} = j\right) = \begin{cases} \left(1 - p_r\right)^2 & x_i = x_{i-1}, y_i = y_{i-1} \\ \left(1 - p_r\right) \cdot p_r & x_i = x_{i-1}, y_i \neq y_{i-1} \quad or \quad x_i \neq x_{i-1}, y_i = y_{i-1} \\ p_r^2 & x_i \neq x_{i-1}, y_i \neq y_{i-1} \end{cases}$$

als eine Rekombinationsübergangsmatrix verwendet wird, wobei $p_r$ re/N ist.

16. Computerlesbares Medium nach Anspruch 15, wobei re 25 ist.

17. Computerlesbares Medium nach Anspruch 14, wobei die Vielzahl von Instruktionen geeignet ist, um das Sequenzierungsergebnis von dem Fötus mit einer Bezugssequenz abzugleichen, um das Sequenzierungsergebnis, das sich von der vorbestimmten Region ableitet, zu bestimmen,
wobei die Vielzahl von Instruktionen ferner geeignet ist, um eine Base zu bestimmen, welche die höchste Wahrscheinlichkeit aufweist, und zwar basierend auf einer Formel

$$P_{i,base} = \sum_{k \in \{0,1\}} \frac{1}{2}(1 - \varepsilon)\Delta\left(base, m_k\right) + \frac{1}{2}\varepsilon \cdot \Delta\left(base, m_{x_i}\right) + \frac{1}{2}\varepsilon \cdot \Delta\left(base, f_{y_i}\right)$$

wobei $\Delta(x, y) = \begin{cases} 1 - e & x = y \\ e/3 & x \neq y \end{cases}$

**Revendications**

1. Procédé de détermination d'informations de base d'une région prédéterminée d'un génome foetal, comprenant les étapes suivantes :

   extraction d'un échantillon d'ADN génomique d'un foetus à partir de sang périphérique d'une femme enceinte, construction d'une bibliothèque de séquençage à partir de l'échantillon d'ADN génomique du foetus ;
   soumission de la bibliothèque de séquençage à un séquençage, pour obtenir un résultat de séquençage du foetus constitué d'une pluralité de données de séquençage ; et
   détermination des informations de base de la région prédéterminée à partir du résultat de séquençage du foetus associé à des informations génétiques d'une personne apparentée à l'aide d'un modèle de Markov caché, dans lequel les informations de base de la région prédéterminée comprennent la recombinaison des haplotypes foetaux la plus possible,
   et les informations de base de la région prédéterminée sont obtenues par :

   construction d'un vecteur de distribution de probabilité d'un état initial et d'une matrice de transition de recombinaison des haplotypes ;
   construction d'une matrice de probabilité des observations ;
   construction d'une matrice de probabilité partielle et d'un curseur d'inversion ;
   détermination d'un état final et retraçage d'un chemin optionnel ; et
   production d'un résultat pour analyser la séquence d'ADN du génome foetal.

2. Procédé selon la revendication 1, dans lequel la bibliothèque de séquençage est soumise à un séquençage par au moins un appareil choisi parmi Illumina-Solexa, ABI-Solid, Roche-454 et un appareil de séquençage monomoléculaire.

3. Procédé selon la revendication 1, comprenant en outre une étape consistant à aligner le résultat de séquençage du foetus avec une séquence de référence, pour déterminer un résultat de séquençage dérivé de la région prédéterminée,
   dans lequel l'étape d'alignement du résultat de séquençage du génome foetal avec la séquence de référence pour déterminer un résultat de séquençage dérivé de la région prédéterminé comprend en outre :

   la détermination d'une base ayant la probabilité la plus élevée à partir de la formule suivante

$$P_{i,base} = \sum_{k \in \{0,1\}} \frac{1}{2}(1-\varepsilon)\Delta\left(base, m_k\right) + \frac{1}{2}\varepsilon \cdot \Delta\left(base, m_{x_i}\right) + \frac{1}{2}\varepsilon \cdot \Delta\left(base, f_{y_i}\right)$$

   dans laquelle

$$\Delta(x,y) = \begin{cases} 1-e & x = y \\ e/3 & x \neq y \end{cases}$$

4. Procédé selon la revendication 3, dans lequel la séquence de référence est un génome de référence humain.

5. Procédé selon la revendication 1, dans lequel la personne apparentée est un parent du foetus.

6. Procédé selon la revendication 1, dans lequel l'étape de détermination des informations de base de la région prédéterminée à l'aide du modèle de Markov caché est effectuée à partir de l'algorithme de Viterbi,
   et dans l'algorithme de Viterbi, 0,25 est utilisé comme distribution de probabilité d'un état initial, re/N est utilisé comme probabilités de recombinaison, re étant égal à 25~30 et N étant une longueur de la région prédéterminée,

$$a_{jk} = \Pr\left(q_i = k \middle| q_{i-1} = j\right) = \begin{cases} (1 - p_r)^2 & x_i = x_{i-1}, y_i = y_{i-1} \\ (1 - p_r) \cdot p_r & x_i = x_{i-1}, y_i \neq y_{i-1} \quad or \quad x_i \neq x_{i-1}, y_i = y_{i-1} \\ p_r^2 & x_i \neq x_{i-1}, y_i \neq y_{i-1} \end{cases}$$

est utilisé comme matrice de transition de recombinaison, où $p_r$ est re/N.

7. Procédé selon la revendication 6, dans lequel re est égal à 25.

8. Procédé selon la revendication 1, dans lequel la région prédéterminée est un site déterminé précédemment comme ayant un polymorphisme génétique,
dans lequel le polymorphisme génétique est au moins un choisi entre un polymorphisme mononucléotidique et STR.

9. Système de détermination d'informations de base d'une région prédéterminée d'un génome foetal, comprenant :

   un appareil d'extraction d'échantillon d'ADN, adapté pour extraire un échantillon d'ADN génomique d'un foetus à partir de sang périphérique d'une femme enceinte,
   un appareil de construction de bibliothèque, adapté pour construire une bibliothèque de séquençage à partir de l'échantillon d'ADN génomique du foetus ;
   un appareil de séquençage, relié à l'appareil de construction de bibliothèque, et adapté pour soumettre la bibliothèque de séquençage à un séquençage, pour obtenir un résultat de séquençage du foetus constitué d'une pluralité de données de séquençage ; et
   un appareil d'analyse, relié à l'appareil de séquençage, et adapté pour déterminer les informations de base de la région prédéterminée à partir du résultat de séquençage du foetus associé à des informations génétiques d'une personne apparentée en utilisant un modèle de Markov caché,
   dans lequel les informations de base de la région prédéterminée comprennent la recombinaison des haplotypes foetaux la plus possible,
   et les informations de base de la région prédéterminée sont obtenues par :

      construction d'un vecteur de distribution de probabilité d'un état initial et d'une matrice de transition de recombinaison des haplotypes ;
      construction d'une matrice de probabilité des observations ;
      construction d'une matrice de probabilité partielle et d'un curseur d'inversion ;
      détermination d'un état final et retraçage d'un chemin optionnel ; et
      production d'un résultat pour analyser la séquence d'ADN du génome foetal.

10. système selon la revendication 9, dans lequel l'appareil de séquençage est au moins un appareil choisi parmi Illumina-Solexa, ABI-Solid, Roche-454 et un appareil de séquençage monomoléculaire.

11. Système selon la revendication 9, comprenant en outre un appareil d'alignement, relié à l'appareil de séquençage, et adapté pour aligner le résultat de séquençage du foetus avec une séquence de référence, pour déterminer un résultat de séquençage dérivé de la région prédéterminée,
dans lequel l'appareil d'alignement est adapté pour déterminer une base ayant la probabilité la plus élevée à partir de la formule suivante

$$P_{i,base} = \sum_{k \in \{0,1\}} \frac{1}{2}(1 - \varepsilon)\Delta\left(base, m_k\right) + \frac{1}{2}\varepsilon \cdot \Delta\left(base, m_{x_i}\right) + \frac{1}{2}\varepsilon \cdot \Delta\left(base, f_{y_i}\right)$$

dans laquelle

$$\Delta(x, y) = \begin{cases} 1 - e & x = y \\ e/3 & x \neq y \end{cases}$$

**12.** Système selon la revendication 9, dans lequel l'appareil d'analyse est adapté pour déterminer les informations de base de la région prédéterminée en utilisant un modèle de Markov caché basé sur l'algorithme de Viterbi, dans lequel dans l'algorithme de Viterbi, 0,25 est utilisé comme distribution de probabilité d'un état initial, re/N est utilisé comme probabilités de recombinaison, re étant égal à 25~30 et N étant une longueur de la région prédéterminée,

$$a_{jk} = \Pr\left(q_i = k \mid q_{i-1} = j\right) = \begin{cases} \left(1-p_r\right)^2 & x_i = x_{i-1}, y_i = y_{i-1} \\ \left(1-p_r\right) \cdot p_r & x_i = x_{i-1}, y_i \neq y_{i-1} \quad or \quad x_i \neq x_{i-1}, y_i = y_{i-1} \\ p_r^2 & x_i \neq x_{i-1}, y_i \neq y_{i-1} \end{cases}$$

est utilisé comme matrice de transition de recombinaison, où $p_r$ est re/N.

**13.** Système selon la revendication 12, dans lequel re est égal à 25.

**14.** Support lisible par ordinateur comprenant une pluralité d'instructions, adaptées pour déterminer des informations de base d'une région prédéterminée à partir d'un résultat de séquençage d'un foetus associé à des informations génétiques d'une personne apparentée en utilisant un modèle de Markov caché qui amène l'appareil à effectuer :

l'extraction d'un échantillon d'ADN génomique d'un foetus à partir de sang périphérique d'une femme enceinte, la construction d'une bibliothèque de séquençage à partir de l'échantillon d'ADN génomique du foetus ;
la soumission de la bibliothèque de séquençage à un séquençage, pour obtenir un résultat de séquençage du foetus constitué d'une pluralité de données de séquençage ; et
la détermination des informations de base de la région prédéterminée à partir du résultat de séquençage du foetus associé à des informations génétiques d'une personne apparentée à l'aide du modèle de Markov caché, dans lequel les informations de base de la région prédéterminée comprennent la recombinaison des haplotypes foetaux la plus possible,
et les informations de base de la région prédéterminée sont obtenues par :

construction d'un vecteur de distribution de probabilité d'un état initial et d'une matrice de transition de recombinaison des haplotypes ;
construction d'une matrice de probabilité des observations ;
construction d'une matrice de probabilité partielle et d'un curseur d'inversion ;
détermination d'un état final et retraçage d'un chemin optionnel ; et
production d'un résultat pour analyser la séquence d'ADN du génome foetal.

**15.** Support lisible par ordinateur selon la revendication 14, dans lequel la pluralité d'instructions sont adaptées pour déterminer les informations de base de la région prédéterminée en utilisant le modèle de Markov caché basé sur l'algorithme de Viterbi, dans lequel dans l'algorithme de Viterbi, 0,25 est utilisé comme distribution de probabilité d'un état initial, re/N est utilisé comme probabilités de recombinaison, re étant égal à 25~30 et N étant une longueur de la région prédéterminée,

$$a_{jk} = \Pr\left(q_i = k \mid q_{i-1} = j\right) = \begin{cases} \left(1-p_r\right)^2 & x_i = x_{i-1}, y_i = y_{i-1} \\ \left(1-p_r\right) \cdot p_r & x_i = x_{i-1}, y_i \neq y_{i-1} \quad or \quad x_i \neq x_{i-1}, y_i = y_{i-1} \\ p_r^2 & x_i \neq x_{i-1}, y_i \neq y_{i-1} \end{cases}$$

est utilisé comme matrice de transition de recombinaison, où $p_r$ est re/N.

**16.** Support lisible par ordinateur selon la revendication 15, dans lequel re est égal à 25.

**17.** Support lisible par ordinateur selon la revendication 14, dans lequel la pluralité d'instructions sont adaptées pour aligner le résultat de séquençage du foetus avec une séquence de référence, pour déterminer un résultat de séquençage dérivé de la région prédéterminée,
dans lequel la pluralité d'instructions sont adaptées en outre pour déterminer une base ayant la probabilité la plus élevée à partir de la formule suivante

$$P_{i,base} = \sum_{k \in \{0,1\}} \frac{1}{2}(1-\varepsilon)\Delta\left(base, m_k\right) + \frac{1}{2}\varepsilon \cdot \Delta\left(base, m_{x_i}\right) + \frac{1}{2}\varepsilon \cdot \Delta\left(base, f_{y_i}\right)$$

dans laquelle

$$\Delta(x,y) = \begin{cases} 1-e & x = y \\ e/3 & x \neq y \end{cases}.$$

Fig.1

Fig.2

## REFERENCES CITED IN THE DESCRIPTION

**Patent documents cited in the description**

- WO 2007092473 A2
- US 20120095697 A1
- WO 2012021149 A1

**Non-patent literature cited in the description**

- **W. TIMP et al.** *Biophysical Journal,* May 2012, vol. 102 (10), L37-L39
- **MINGJIE LIN et al.** International Conference on Reconfigurable Computing and FPGAs, Reconfig '09. IEEE, 2009, 95-100
- **KUO-CHING LIANG et al.** *IEEE/ACM TRANSACTIONS ON COMPUTATIONAL BIOLOGY AND BIOINFORMATICS,* July 2007, vol. 4 (3), 430-440
- **KUO-CHING LIANG et al.** 40th Annual Conference on Information Sciences and Systems. IEEE, 2006, 1599-1604
- **METZKER ML.** Sequencing technologies-the next generation. *Nat Rev Genet.,* January 2010, vol. 11 (1), 31-46
- **RUSK, NICOLE.** Cheap Third-Generation Sequencing. *Nature Methods,* 01 April 2009, vol. 6 (4), 244-245
- **LAWRENCE R. RABINER.** *PROCEEDINGS OF THE IEEE,* February 1989, vol. 77 (2
- **J. SAMBROOK et al.** Molecular Cloning: A Laboratory Manual. Science Press